(19) Europäisches Patentamt
European Patent Office
Office européen des brevets

(11) Publication number: **0 289 951**
**A2**

(12) # EUROPEAN PATENT APPLICATION

(21) Application number: 88106906.6

(51) Int. Cl.4: C01B 17/02

(22) Date of filing: 29.04.88

A request for correction has been filed pursuant to Rule 88 EPC. A decision on the request will be taken during the proceedings before the Examining Division (Guidelines for Examination in the EPO, A-V, 2.2).

(30) Priority: 08.05.87 US 47955

(43) Date of publication of application:
09.11.88 Bulletin 88/45

(84) Designated Contracting States:
BE DE FR GB IT NL SE

(71) Applicant: PENNWALT CORPORATION
Pennwalt Building Three Parkway
Philadelphia Pennsylvania 19102(US)

(72) Inventor: Carroll, Glenn Thomas
2437 Chestnut Avenue
Jeffersonvile Pennsylvania 19403(US)
Inventor: Lindstrom, Michael Jeffrey
425 Willow Circle
Downingtown Pennsylvania 19335(US)

(74) Representative: Kraus, Walter, Dr. et al
Patentanwälte Kraus, Weisert & Partner
Thomas-Wimmer-Ring 15
D-8000 München 22(DE)

(54) **Sulfur dissolving composition.**

(57) A composition, useful for dissolving sulfur, comprising a disulfide, polysulfide and mixtures thereof containing a catalytic amount of a mixture of a basic nitrogen-containing compound and an alcoholic or aqueous solution of an alkali hydroxide, alkoxide, or carbonate and methods for using the composition to prevent sulfur-plugging in a system, are disclosed.

EP 0 289 951 A2

# Sulfur Dissolving Composition

## Background

This invention relates to a composition of matter which is a liquid disulfide, polysulfide or mixture of these incorporating a catalytic amount of a mixture of a basic nitrogen-containing compound and alcoholic or aqueous solution of an alkali hydroxide, alkoxide, or carbonate to provide a substance capable of dissolving unexpectedly large amounts of sulfur. It also relates to a process for dissolving sulfur utilizing such composition. Additionally, it relates to a process for preparing polysulfides by reacting a disulfide or low rank polysulfide with sulfur in the presence of the above-defined catalyst system.

In the processing of sour gas wells, sulfur may form deposits that can plug the well and cease production. These deposits have been prevented or dissolved by flowing solvents such as carbon disulfide, organic solvents, and aqueous alkylamines, downhole to dissolve the sulfur plug. The solvent is injected downhole and the well is allowed to soak for a sufficient mount of time to dissolve any existing sulfur plugs. Alternatively, the solvent can be injected continuously in amounts sufficient to prevent the formation of sulfur deposits. The above systems all have various disadvantages such as toxicity, flammability, corrosivity, or limited ability to dissolve sulfur.

## Prior Art

Dialkyl disulfides, either alone or blended with dialkyl sulfides (US 3,531,160), have become the sulfur solvents of choice. Hyne [Alberta Sulfur Research Ltd. (ASRL), Quarterly Bulletin, Vol. XVIII, Nos. 2, 3, and 4, 1982, p. 44] has shown that lower dialkyl disulfides, especially dimethyl disulfide (DMDS) are preferred. Alone, the disulfides take up only a limited amount of sulfur; however, in conjunction with a suitable catalyst system, they take up approximately 1.5 times their weight in sulfur at room temperature.

Sharp and Sudduth (US 3,846,311) teach that a composition of one or more dialkyl disulfides and an unsubstituted saturated aliphatic amine (up to 10 wt%) is capable of consuming over 200 wt% sulfur after the composition has aged. However, in French Patents FR 2,152,532 and FR 2,159,320, similar compositions are disclosed to be useful without aging. It is also taught in the art that if a small amount of sulfur (5-40 wt%) is added to the above compositions, the rate of sulfur uptake is accelerated (US 4,239,630).

Sharp and Yarborough (US 4,290,900) teach that the above sulfide-amine composition is not as effective if vaporization is possible as occurs, for example, in deep wells, where temperatures greater than 250°F may be reached. Therefore, they disclose using a composition of a dialkyl disulfide and a fatty acid amine (>30 wt%) which also has been aged. Further, the art teaches that the addition of 60 wt% sulfur to the above composition accelerates sulfur uptake(US 4,248,717).

Hyne and coworkers (ASR Quarterly Bulletin, Vol. XIX, Nos. 1 & 2, 1982, p. 4) show that sodium hydrosulfide (NaSH) and dimethylformamide (used as a co-solvent) is an effective system for catalyzing sulfur uptake by dimethyl disulfide. They also demonstrate that a variety of alkali salts of a series of thiophenols, in conjunction with dimethylformamide, catalyzes sulfur-uptake. It is known that the sulfur recovery systems of Hyne et al. have one major disadvantage; they are unstable and lose activity within 3-10 days upon standing at room temperature.

## Statement of the Invention

This invention is a composition comprising a disulfide, a polysulfide or mixture thereof and a catalytic amount of a mixture of a basic nitrogen-containing compound and an aqueous or alcoholic solution of an alkali hydroxide, alkoxide, or carbonate. The disulfide and polysulfide have the following formula:

$RSS_{\underline{a}}SR^1$

wherein R and $R^1$ are independently alkyl, aryl, alkaryl, alkoxyalkyl, or hydroxyalkyl wherein the alkyl moiety has from 1 to 24 carbon atoms and $\underline{a}$ is 0-3. The value of $\underline{a}$ represents the average number of internal sulfurs in the composition and not the maximum number of sulfurs for any one species in the composition.

This invention also includes a process for the use of said composition in a system, such as oil and gas wells, to dissolve sulfur plugs or prevent their formation, or in a pipe or other equipment whose operation may be hindered or prevented due to sulfur buildup.

Finally, this invention includes a process for preparing polysulfides by reacting a disulfide or low rank poly sulfide with sulfur in the presence of one or more of the aforementioned catalyst compositions.

## Detailed Description of the Invention

This invention is a superior composition for dissolving sulfur. The composition may be used whenever a requirement for sulfur removal exists. One such application is to dissolve or prevent sulfur plugs in sour and super-sour oil and gas wells.

A disulfide or a polysulfide of a low sulfur rank can be used for the composition of this invention. The sulfur rank is defined as the average of the number of sulfur atoms between the two alkyl groups in a mixture of di-and polysulfides. A rank greater than 2 but less than 3 is considered low. A low sulfur rank is preferred since a polysulfide with a sulfur rank greater than 3 will have a limited capacity to take up additional sulfur.

The sulfur is not merely physically dissolved by the compositions of this invention, but a chemical reaction occurs between the disulfide and the sulfur, which results in the insertion of the sulfur into the sulfur-sulfur bond of the disulfide and produces a polysulfide of higher sulfur rank.

The preferred disulfide or polysulfide component of the composition of this invention has the formula

$RSS_{a}SR^1$

wherein R and $R^1$ are $C_1$-$C_{24}$ alkyl radicals and a ranges from 0 to 3, more preferably where R and $R^1$ are alkyl radicals having from 1 to 4 carbon atoms and a ranges from 0 to 1.5. A further preference is a di-or polysulfide where R and $R^1$ are methyl and a ranges from 0 to 1.5, while in the most preferred embodiment, dimethyl disulfide is used.

The basic nitrogen-containing compounds of the catalyst mixture include, for example, ammonia, aliphatic amines (e.g. cyclohexylamine), aryl amines (e.g. aniline), alkaryl amines (e.g. toluidine), poly-(alkyleneoxy)alkanolamines and/or their respective ethers, polyalkyleneoxyamines and/or polyamines, amides (e.g. dimethylformamide), sulfenamides (e.g. n-cyclohexyl-2-benzothiazolesulfenamide), imines (e.g. cyclohexanoneimine), and/or enamines (e.g. 1-dimethylaminocyclohexene). Said nitrogen-containing compounds can range from an amount sufficient to improve the sulfur dissolving ability of the di-or polysulfide up to 10 wt%. Amounts as low as 0.1 wt% are useful although much smaller proportions should also be effective. Amounts as low as 5 parts/million have been found to be effective in similar systems.

A preferred basic nitrogen-containing compound is an amide, more preferably, dimethylformamide. Another preferred compound is a sulfenamide, more preferably, N-cyclohexyl-2-benzothiazolsulfenamide. Still other preferred nitrogen-containing compounds are aliphatic amines, e.g., triethylamine, alkanolamines, e.g., dimethylaminoethanol, polyalkyleneoxyamines and polyalkyleneoxypolyamines.

The formulas 1, 2, 3, and 4 shown below are given as examples to demonstrate the types of polyalkyleneoxyamines and -polyamines that will act as catalysts for sulfur-uptake by disulfides or polysulfides of low sulfur rank.

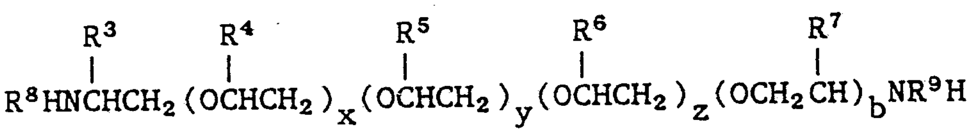
(1)

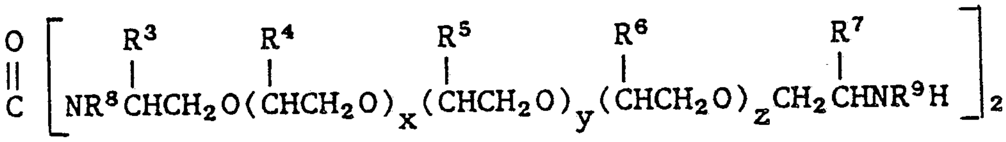
(2)

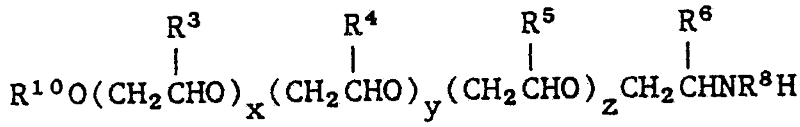
(3)

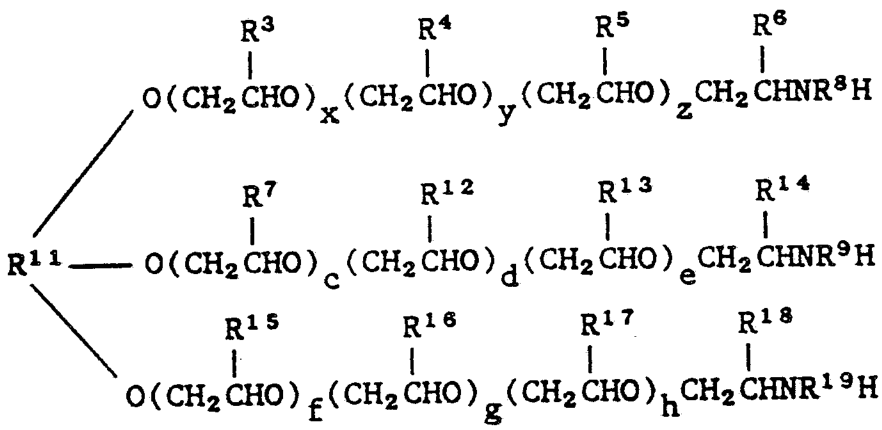
(4)

where $R^3$, $R^4$, $R^5$, $R^6$, $R^7$, $R^{10}$, $R^{12}$, $R^{13}$, $R^{14}$, $R^{15}$, $R^{16}$, $R^{17}$, and $R^{18}$ are independently H, alkyl, alkaryl, hydroxyalkyl, alkoxyalkyl, haloalkyl, wherein the alkyl moieties have from 1 to 20 carbon atoms, or phenyl; $R^8$, $R^9$, and $R^{19}$ are independently H, alkyl, hydroxyalkyl, alkoxyalkyl, alkaryl wherein the alkyl moieties have from 1 to 10 carbon atoms, aryl, or $CONH_2$; $R^{11}$ is the hydrocarbon residue of a triol; and b, c, d, e, f, g, h, x, y, and z are independently values of 0-200, provided, however, that the total of such values is no less than 2.

Jeffamines®, a series of polyalkyleneoxyamines produced by the Texaco Chemical Company, are but one example encompassed by the above formulas. Furthermore, any polyalkylene-oxy-compound which contains an amine functionality will be active. Additionally, formula 4 shows the hydrocarbon residue of a triol, such as glycerol, ($R^{11}$) as the base of the compound, although any other similar polyalkyleneoxyamine which incorporates any polyol as its base should also be effective.

Examples of Jeffamine® products which are preferred for this invention include those identified below under the alpha-numeric product designation.

C-346 = 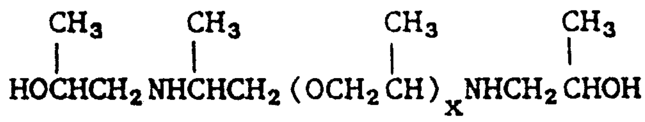 x = 2.6

T-403 = 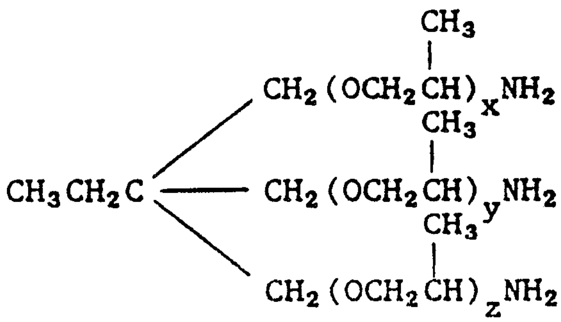 x + y + z ≃ 5.3

T-3000 = 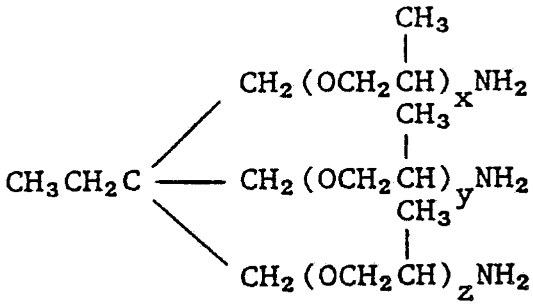 Avg. Molecular Wt.=3000

D-230 = 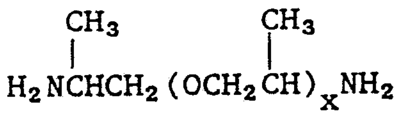 x = 2.6

D-400 = 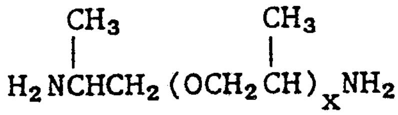 x = 5.6

D-2000 = 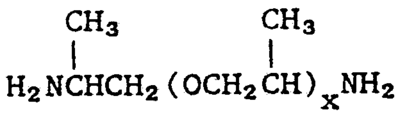 x = 33.1

ED600= 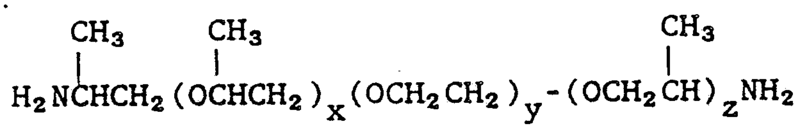 x+z = 2.5, y = 8.5

ED2001= 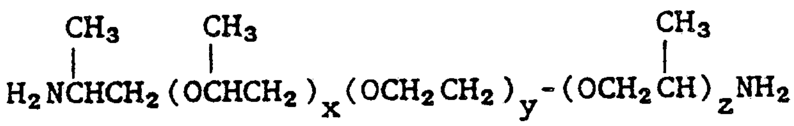 x+z = 2.5, y = 40.5

ED4000= 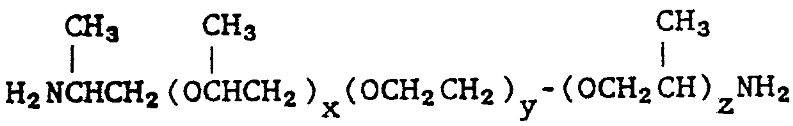 x+z = 2.5, y = 86.0

ED6000= 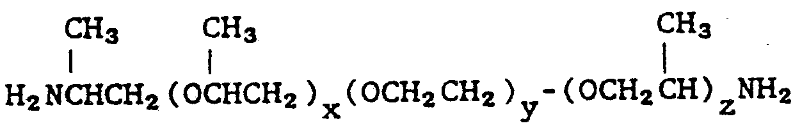 x+z = 2.5, y = 131.5

EDR-148= $H_2NCH_2CH_2OCH_2CH_2OCH_2CH_2NH_2$

EDR-192= $H_2NCH_2CH_2OCH_2CH_2OCH_2CH_2OCH_2CH_2NH_2$

M-600= 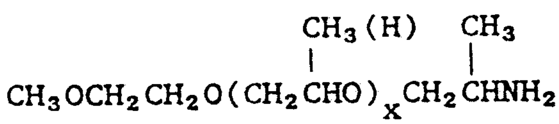 Avg. Molecular Wt.= 600
$CH_3/H \simeq 9$

$CH_3(H)$ $CH_3$

M-1000= $CH_3OCH_2CH_2O(CH_2CHO)_xCH_2CHNH_2$ Avg. Molecular Wt.=1000
$CH_3/H \cong 0.17$

$CH_3(H)$ $CH_3$

M-2005= $CH_3OCH_2CH_2O(CH_2CHO)_xCH_2CHNH_2$ Avg. Molecular Wt.=2000
$CH_3/H \cong 16$

$CH_3(H)$ $CH_3$

M-2070= $CH_3OCH_2CH_2O(CH_2CHO)_xCH_2CHNH_2$ Avg. Molecular Wt.= 2000
$CH_3/H \cong 0.32$

MNPA= 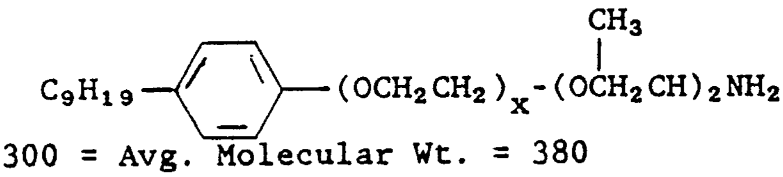

MNPA-300 = Avg. Molecular Wt. = 380

MNPA-510 = Avg. Molecular Wt. = 510

MNPA-750 = Avg. Molecular Wt. = 750

The catalyst mixture of this invention contains an alcoholic or aqueous solution of an alkali metal or a alkaline earth metal hydroxide, alkoxide or carbonate. Preferably, for solubility reasons, the alkali metal compounds are employed.

The preferred catalyst mixture contains an alcoholic solution of an alkali hydroxide, alkoxide or carbonate more preferably, a $C_1$-$C_4$ alkanolic solution of sodium or potassium hydroxide or alkoxide. Most preferably, the basic alcoholic solution is a methanolic solution of sodium or potassium hydroxide or methoxide.

The preferred concentration of the alkali hydroxide, alkoxide, or carbonate in the alcohol or water is from 1 wt% to saturated. The preferred concentration ranges of the alcoholic solution of an alkali hydroxide alkoxide or carbonate and said nitrogen-containing compound are governed by the requirements of activity and cost of the composition, since, as the concentration of these species increases, so does the composition's cost and rate of sulfur-uptake. Both the alcoholic solution of an alkali hydroxide, alkoxide, and/or carbonate and said nitrogen-containing compound are active catalysts at levels as low as 5 ppm.

The concentration of the alcoholic solution of an alkali hydroxide, alkoxide, and/or carbonate and the nitrogen-containing compound are further controlled by the requirements for sulfur capacity. The disulfide and/or polysulfide consumes the sulfur while the alcoholic solution of an alkali hydroxide, alkoxide, and/or carbonate and said nitrogen-containing compound act as catalysts to initiate the process. Therefore, their concentrations will be dictated by the requirement for either a composition which dissolves a smaller amount of sulfur quickly, or a larger amount of sulfur more slowly.

The concentration of the alcoholic solution of an alkali hydroxide, alkoxide, and/or carbonate is still further restricted if the requirement of a homogeneous solution exists since greater concentrations result in some precipitation of the alkali hydroxide, alkoxide or carbonate. The addition of $H_2S$ causes the dissolution of the precipitate.

The compositions of this invention may require, or have their activity enhanced, by activation with $H_2S$ or an alkyl, alkaryl, aryl, hydroxyalkyl or an alkoxyalkyl mercaptan wherein the alkyl moiety has from 1 to 24 carbon atoms. Said activation is possible without pretreatment with $H_2S$ since it is well known that sour and super-sour gas and oil wells, which plug with sulfur, contain $H_2S$. The use of said compositions in other applications where $H_2S$ is not present, however, may require pretreatment with $H_2S$ and/or said mercaptan to dissolve sulfur or enhance the rate of sulfur-uptake. If activation by $H_2S$ and/or a volatile mercaptan is desired, then the activated composition can optionally be subsequently purged with nitrogen to remove residual $H_2S$ and/or mercaptan. The amount of $H_2S$ and/or said mercaptan may range from an amount large enough to be effective to 10 wt%. Amounts as small as 0.05 wt% have been found to be effective.

## Examples

### Example 1

The compositions of dimethyl disulfide, an amine, and NaOH or $NaOCH_3$ in methanol, shown in Table 1, were bubbled with $H_2S$ for 3 minutes to simulate sour gas well conditions, and thereafter, 3.5 g of sulfur were added at room temperature; the times were noted for the consumption of the sulfur. The superior sulfur-dissolving power of the compositions of

Table 1

| DMDS (grams) | Amine (milligrams) | 15% NaOH/Methanol (milligrams) | Time (minutes) |
|---|---|---|---|
| 9.5 g | Triethylamine (4.7 mg) | 75.1 mg | 8.50 |
| 9.5 g | Jeffamine ED-600 (23.8 mg) | 26.6 mg | 1.12 |

A mixture of sodium hydrosulfide (NaSH) and dimethylformamide (DMF) was prepared by adding the NaSH (0.015g) to DMF (0.5 g) which had been first degassed with nitrogen. The resulting green mixture was then added to DMDS (9.5 g) after stirring for 1 hour. Sulfur (3.5 g) was subsequently added to the DMDS/DMF/NaSH mixture and a time of 10.17 minutes was noted for its consumption. Hyne reports a value of 5.83 minutes to take up 1 g of sulfur using the same composition. (ASRI Quarterly Bulletin, Vol. XIX, Nos. 1 & 2, 1982, p. 4). This example serves as a standard prior art reference with which to compare the compositions of this invention. This prior art formulation was not bubbled with $H_2S$ (Hyne also reports that $H_2S$ is not required since the mixture contains NaSH).

## Claims

1. A composition comprising a major amount of a disulfide, polysulfide, or mixture thereof, having the following formula:

$RSS_aSR^1$

where R and $R^1$ are independently alkyl, aryl, alkaryl, alkoxyalkyl, or hydroxyalkyl radicals wherein the alkyl moiety has from 1 to 24 carbon atoms and a is 0 to 3, and a catalytic amount of a mixture of an aqueous or alcoholic solution of an alkali hydroxide, alkoxide, or carbonate and one or more basic nitrogen-containing compounds.

2. The composition of claim 1 where R and $R^1$ are alkyl radicals.

3. The composition of claim 2 where R and $R^1$ are $C_1$-$C_4$ alkyl radicals and a is 0 to 1.5.

4. The composition of claim 3 where R and $R^1$ are methyl.

5. The composition of claim 4 where a is 0.

6. The composition of claim 1 where the basic nitrogen-containing compound ranges in concentration fro 5 parts per million to 10 wt%, based on the weight of the composition.

7. The composition of claim 1 where the basic nitrogen-containing compound is an amide.

8. The composition of claim 7 where the amide is dimethyl-formamide.

9. The composition of claim 1 where the basic nitrogen-containing compound is a sulfenamide.

10. The composition of claim 9 where the sulfenamide is N-cyclohexyl-2-benzothiazolesulfenamide.

11. The composition of claim 1 where the basic nitrogen-containing compound is an alkanolamine.

12. The composition of claim 11 where the alkanolamine is dimethylaminoethanol.

13. The composition of claim 1 where the basic nitrogen-containing compound is an aliphatic amine.

14. The composition of claim 13 where the aliphatic amine is triethylamine.

15. The composition of claim 1 where the basic nitrogen-containing compound is a polyalkyleneoxyamine or polyamine.

16. The composition of claim 15 where the polyalkylene-oxyamine or polyamine is a compound of the following formula

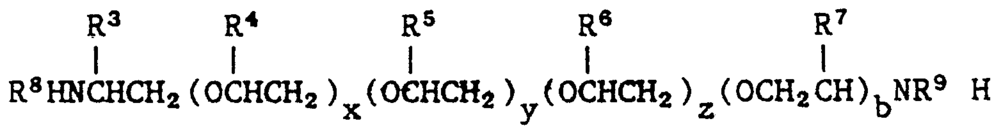

where $R^{3-7}$ are independently H, alkyl, alkaryl, hydroxyalkyl, alkoxyalkyl, haloalkyl wherein the alkyl moieties have from 1 to 20 carbon atoms, or phenyl; $R^{8-9}$ are inde-pendently H, alkyl, hydroxyalkyl, alkoxyalkyl, alkaryl wherein the alkyl moieties have from 1 to 10 carbon atoms, aryl or $-CONH_2$; and b, x, y and z are independently values of 0 to 200 provided, however, that the total of such values is no less than 2.

17. The composition of claim 15 where the polyalkyleneoxyamine or polyamine is a compound of the following formula

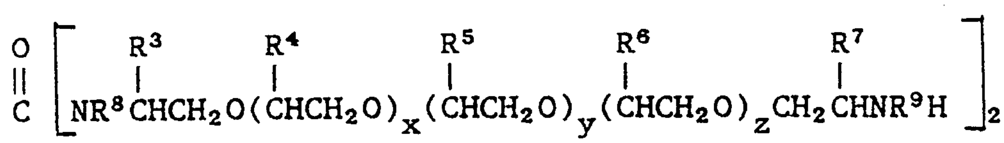

where $R^{3-7}$ are independently H, alkyl, alkaryl, hydroxyalkyl, alkoxyalkyl, haloalkyl wherein the alkyl moieties have from 1 to 20 carbon atoms, or phenyl; $R^{8-9}$ are independently H, alkyl, hydroxyalkyl, alkoxyalkyl, alkaryl wherein the alkyl moieties have from 1 to 10 carbon atoms, aryl or $-CONH_2$; and x, y and z are independently values of 0 to 200 provided, however, that the total of such values is no less than 2.

18. The composition of claim 15 where the polyalkyleneoxyamine or polyamine is a compound of the following formula

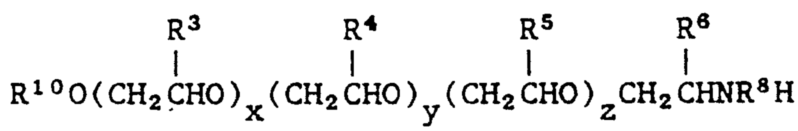

where $R^{3-6}$ and $R^{10}$ are independently H, alkyl, alkaryl, hydroxyalkyl, alkoxyalkyl, haloalkyl wherein the alkyl moieties have from 1 to 20 carbon atoms, or phenyl; $R^8$ is H, alkyl, hydroxyalkyl, alkoxyalkyl, alkaryl, wherein the alkyl moieties have from 1 to 10 carbon atoms, aryl or $-CONH_2$; and x, y and z are independently values of 0 to 200 provided, however, that the total of such values is no less than 2.

19. The composition of claim 15 where the polyalkyleneoxyamine or polyamine is a compound of the following formula:

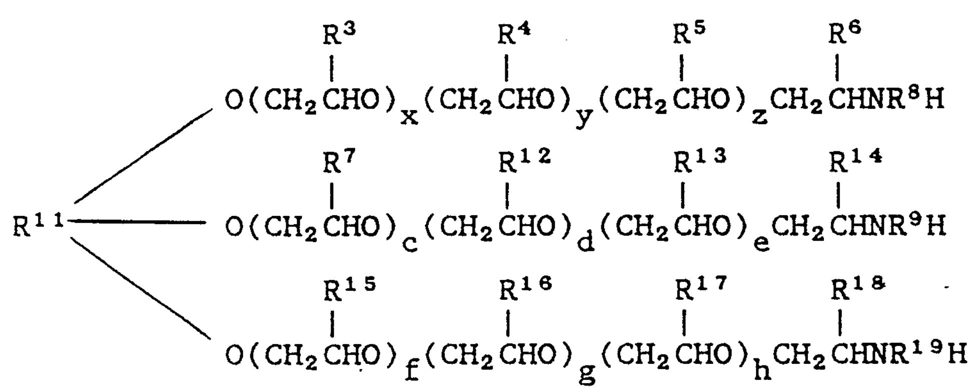

where $R^{3-7}$ and $R^{12-18}$ are independently H, alkyl, alkaryl, hydroxyalkyl, alkoxyalkyl, haloalkyl wherein the alkyl moieties have from 1 to 20 carbon atoms, or phenyl; $R^{8-9}$ and $R^{19}$ are independently H, alkyl, hydroxyalkyl, alkoxy-alkyl, alkaryl, wherein the alkyl moieties have from 1 to 10 carbon atoms, aryl or $-CONH_2$; $R^{11}$ is the hydrocarbon residue of a triol; and c, d, e, f, g, h, x, y and z are independently values of 0 to 200 provided, however, that the total of such values is no less than 2.

20. The composition of claim 1 where the concentration of the aqueous or alcoholic solution of an alkali hydroxide or alkoxide, or carbonate ranges from 5 parts (wt.) per million to 10% based on the weight of the composition.

21. The composition of claim 20 where the aqueous or alcoholic solution of the alkali hydroxide is a $C_1$-$C_4$ alkanolic solution of sodium or potassium hydroxide or alkoxide.

22. The composition of claim 21 where the aqueous or alcoholic solution of the alkali hydroxide is a methanolic solution of sodium or potassium hydroxide or alkoxide.

23. The composition of claim 1 treated with hydrogen sulfide in an amount sufficient to activate said composition to dissolve sulfur and up to about 10% based on the weight of said composition.

24. The composition of claim 23 which is purged with an inert gas to remove residual hydrogen sulfide subsequent to treatment therewith.

25. The composition of claim 1 treated with a alkyl, aryl, alkaryl, hydroxyalkyl, or alkoxyalkyl mercaptan, having from 1 to 24 carbon atoms in the alkyl moieties, in an amount sufficient to activate said composition to dissolve sulfur and up to about 10% based on the weight of said composition.

26. The composition of claim 25 which is purged with an inert gas to remove residual mercaptan subsequent to treatment therewith.

KRAUS · WEISERT & PARTNER
PATENTANWÄLTE
UND
ZUGELASSENE VERTRETER VOR DEM EUROPÄISCHEN PATENTAMT

Patentanwälte Thomas-Wimmer-Ring 15 D-8000 München 22

DR. WALTER KRAUS
DIPLOMCHEMIKER
DR.-ING. ANNEKÄTE WEISERT
DIPL.-ING.
JOHANNES SPIES
DIPL.-PHYS.

THOMAS-WIMMER-RING 15
D-8000 MÜNCHEN 22
TELEFON (0 89) 22 73 77
TELEX 5-212 156 kpat d
TELEFAX (0 89) 22 79 94

European Patent Office

8000 Munich 2

EPA - EPO - OEB
MÜNCHEN
Empfang bestätigt
Receipt acknowledged
Accusé reception
SD

UNSERE NR.: 6449 AW/pl
OUR FILE:
Bitte in der Antwort stets angeben
Please refer to in your reply

EPA EPO OEB
DG 1
Reçu:
16 -05- 1988
06 ANL. ZEICHN.

Re: European patent application 88 106 906.6
Pennwalt Corporation

Enclosed is a new page 12. It was found that the last sentence on page 12, line 25, was incomplete and should read as follows: "The superior sulfur-dissolving power of the compositions of this invention, compared to the prior art standard (discussed in the following table 1) is clearly shown by this data."
It is politely submitted that the amendment is allowable under Rule 88 EPC. Rule 88 EPC states that errors in any document filed with the European Patent Office may be corrected on request, if the correction is obvious in the sense that it is immediately evident that nothing else would have been intended then what is offered as the correction.

European Patent Attorney

encl.:
new page 12, in triplicate